# EUROPEAN PATENT APPLICATION

(11) **EP 3 603 702 A1**
(43) Date of publication of application: **05.02.2020**
(21) Application number: 18844414.5
(22) Date of filing: 04.01.2018
(51) Int. Cl.: A61M 5/20, A61M 5/31

(54) **INJECTION PEN CAPABLE OF FINE ADJUSTMENT**

(30) Priority: 08.08.2017 CN 201710671909; 08.08.2017 CN 201720979435 U
(71) Applicant: Gansu Changee Bio-pharmaceutical Co., Ltd., Tianshui, Gansu 741020 (CN)
(72) Inventor: HAN, Yitao, Tianshui Gansu 741020 (CN); PAN, Junfeng, Tianshui Gansu 741020 (CN); LI, Guoan, Tianshui Gansu 741020 (CN)
(74) Representative: Schollweck, Susanne
(86) International application number: PCT/CN2018/071357
(87) International publication number: WO 2019/029123

(57) **Abstract**

An injection pen capable of fine adjustment, comprising: a differential spiral transmission mechanism, comprising a push rod (18), a push rod nut (22), a positioning rotary ratchet (21), an adjustment rod (17), and a ratchet tube (8); a dose adjustment mechanism, comprising a knob (2), a numerical code tube (15), an adjustment tube (7), a ratchet connection ring (9), and an adjustment and connection tube (6); a housing (3), the differential spiral transmission mechanism being provided in the housing (3), a window (4) being provided on the housing (3), and a pen core holder (24) being provided in the housing (3); and an injection mechanism, comprising a button connection member (16), a button (1), and a button spring (14). During adjustment, a contact portion between the ratchet connection ring (9) and the ratchet tube (8) clicks under the action of the ratchet so as to prompt a user. In addition, a visual prompt on the window (4) also facilitates the adjustment of a dose. The injection pen capable of fine adjustment can also be reused, and can improve injection precision by converting a spiral motion into a linear motion.

## Description

The present application claims the benefit of priorities to the following two Chinese Patent Applications, the entire disclosures of which are incorporated herein by reference:
(1) Chinese Patent Application No. 201710671909.3, titled "INJECTION PEN CAPABLE OF FINE ADJUSTMENT", filed on August 08, 2017 with the China National Intellectual Property Administration; and
(2) Chinese Patent Application No. 201720979435.4, titled "INJECTION PEN CAPABLE OF FINE ADJUSTMENT", filed on August 08, 2017 with the China National Intellectual Property Administration.

### FIELD

The present application relates to the technical field of medical instruments, in particular to an injection pen capable of fine adjustment.

### BACKGROUND

Conventional drug injection generally requires a professional medical staff to use a disposable sterile syringe for injection, which inevitably causes problems such as glass debris and waste of drug liquid.

In order to solve the above problems, an injection pen capable of self-injection thereby has emerged. When the injection is performed, a self-injection can be realized by fitting a medicine bottle into the injection pen and connecting a matching needle to the injection pen. The entire injection process does not generate glass debris, and the drug liquid does not transfer and is not exposed to the air, thus the safety of the medication can be ensured and the convenience thereof can be improved.

Such self-injection pen can be applied to various kinds of first-aid and chronic diseases such as diabetes, and can help patients with cardiovascular and cerebrovascular diseases and patients with hypoglycemia to achieve self-injection, and further can be applied to first-aid medication for acute poisoning patients or for the above diseases, to hemostasis and pain relief, and to self-rescue for biological and chemical weapons. However, it is inconvenient for a current injection pen to adjust the dosage, and it is difficult to precisely control the dosage during the injection.

Therefore, a technical problem for those skilled in the art to be solved presently is to provide an injection pen capable of fine adjustment for facilitating the adjustment of the dosage and improving the accuracy of the dosage.

### SUMMARY

The present application aims to provide an injection pen capable of fine adjustment, which can effectively solve the problem of inaccurate adjustment of medication dosage.

In order to solve the above technical problem, the following technical solutions are provided according to the present application.

An injection pen capable of fine adjustment includes:
a differential screw drive mechanism, including a push rod for pushing a cork of a cartridge bottle, a push rod nut is screwed to the push rod, and a positioning rotary ratchet axially abutting the push rod nut, an adjusting rod, one end of which is in a detachable connection with the positioning rotary ratchet, and a ratchet tube which controls the rotation of the adjusting rod, and the ratchet tube is arranged around an outside of the adjusting rod;
a dose adjustment mechanism, including a knob, a numerical code tube, an adjusting tube, a ratchet connecting ring, and an adjustment connecting tube, wherein the ratchet connecting ring is arranged around the outside of the adjusting rod, the adjustment connecting tube is arranged around an outside of the adjusting rod, the ratchet connecting ring and the ratchet tube, ratchets at two ends of the ratchet connecting ring are inclined in a same direction, the two ends of the ratchet connecting ring are respectively connected to the adjusting rod and the ratchet tube by the ratchets, the adjustment connecting tube is in a threaded connection with the outside of the adjusting tube, the numerical code tube is arranged around the outside of the adjustment connecting tube, and a top end of the numerical code tube is snapped inside the knob, and an outer wall of the numerical code tube is provided with scale marks distributed in a spiral shape;
a housing, wherein the differential screw drive mechanism is arranged in the housing, the housing is provided with a window for displaying a scale on the numerical code tube and a pen core holder for mounting the cartridge bottle; and
an injection mechanism, including a button connecting member which has one end in contact with one end of the ratchet tube, a button which pushes the button connecting member to move axially, and a button spring which abuts the button connecting member and the button at two ends, wherein the button connecting member and the ratchet connecting ring move axially, and the numerical code tube move spirally in an axial direction, after the button is pressed.

In an embodiment, a return spring is provided in the housing for pushing the push rod nut to move downward and further driving the positioning rotary ratchet and the adjusting rod to be separated from each other. A bottom end of the return spring closely abuts against an upper end of the push rod nut.

In an embodiment, the housing is further provided with a positioning ratchet. The positioning ratchet is arranged around outside of the adjusting rod, and an injection voicing ratchet and an injection bouncing spring are arranged around the outside of the adjusting rod. The injection voicing ratchet butts the positioning ratchet through the injection bouncing spring.

In an embodiment, a pusher head is provided between the push rod nut and the cork of the cartridge bottle.

In an embodiment, axially distributed grooves are provided on an outside wall of the adjusting rod, and sliding blocks cooperating with the grooves are provided on an inside wall of the ratchet tube.

In an embodiment, the window is provided with a window cover.

Compared with the conventional technology, the technical solution according to the present application has the following advantages.

The injection pen capable of fine adjustment according to the present application includes: the differential screw drive mechanism, comprising the push rod for pushing the cork of the cartridge bottle, the push rod nut screwed to the push rod, and the positioning rotary ratchet axially abutting the push rod nut, the adjusting rod, one end of which is in a detachable connection with the positioning rotary ratchet, and the ratchet tube which controls the rotation of the adjusting rod, wherein the ratchet tube is arranged around the outside of the adjusting rod; the dose adjustment mechanism, comprising the knob, the numerical code tube, the adjusting tube, the ratchet connecting ring, and the adjustment connecting tube, wherein the ratchet connecting ring is arranged around outside of the adjusting rod, the adjustment connecting tube is arranged around outside of the adjusting rod, the ratchet connecting ring and the ratchet tube, ratchets at two ends of the ratchet connecting ring are inclined in a same direction, the two ends of the ratchet connecting ring are respectively connected to the adjusting rod and the ratchet tube by the ratchets, the adjustment connecting tube is in threaded connection with outside of the adjusting tube, the numerical code tube is arranged around outside of the adjustment connecting tube, and the top end of the numerical code tube is snapped inside the knob, and the outer wall of the numerical code tube is provided with scale marks distributed in the spiral shape; the housing, wherein the differential screw drive mechanism is arranged in the housing, the housing is provided with the window for displaying a scale on the numerical code tube and the pen core holder for holding the cartridge bottle; the injection mechanism, comprising the button connecting member which has one end in contact with one end of the ratchet tube, the button which pushes the button connecting member to move axially, and the button spring which abuts the button connecting member and the button at two ends.

When the cartridge bottle is loaded into the pen core holder, the push rod nut is forced to slide upwards, and the positioning rotary ratchet moves accordingly. After the cartridge is fitted in position, the positioning rotary ratchet engages with a ratchet at the end of the adjusting rod. When adjusting the dose before the injection, the knob is rotated to drive the numerical code tube, the adjusting tube, the ratchet connecting ring, the ratchet tube, the button connecting member, and the button to simultaneously move upward, wherein the adjusting tube rotates along a screw lead, the adjusting tube and the ratchet connecting ring are assembled with each other by ratchets, and due to a reaction force from the button spring, there will be "click" sounds at a contact region of the ratchet connecting ring and the ratchet tube, and the adjusted dose can be observed through the window during the adjusting process. If the dose is too much, the dose can be readjusted by turning the knob. Since the ratchet connecting ring has ratchets at both ends, there will also be the "click" sounds at a contact region of the ratchet connecting ring and the adjusting tube. The sounds can remind the user, and the visual prompt of the window can also be combined to facilitate the adjustment of the dose.

After the dose is adjusted to a required amount, the injection can be performed: the button is pressed, the button connecting member moves linearly along its axial direction and drives the ratchet tube, the ratchet connecting ring, the adjusting tube and the numerical code tube, the adjusting rod, and the positioning rotary ratchet to move spirally, which in turn drives the push rod to rotate. Under the action of the push rod nut, the push rod moves axially, and then pushes the cork of the cartridge bottle to inject. The injection pen capable of fine adjustment can be reused, thereby avoiding use of conventional disposable pen supplies and reducing medical waste. In addition, compared with the conventional direct-push injection pen, the injection precision can be effectively improved by converting the spiral motion into the linear motion.

### BRIEF DESCRIPTION OF THE DRAWINGS

In order to illustrate technical solutions in the embodiments of the present application or in the conventional technology more clearly, drawings used in the description of the embodiments or the conventional technology are introduced briefly hereinafter. Obviously, the drawings described hereinafter merely illustrate some embodiments of the present application, and other drawings may be obtained by those skilled in the art based on these drawings without any creative efforts.
Figure 1 is a cross-sectional structural view of an injection pen capable of fine adjustment according to a specific embodiment of the present application;
Figure 2 is a schematic exploded view of the injection pen capable of fine adjustment according to a specific embodiment of the present application; and
Figure 3 is a partial cross-sectional structural view of the injection pen capable of fine adjustment according to a specific embodiment of the present application.

**Reference numerals in the drawings:**

| | |
|---|---|
| 1 button, | 2 knob, |
| 3 housing, | 4 window, |
| 5 window cover, | 6 adjustment connecting tube, |
| 7 adjusting tube, | 8 ratchet tube, |
| 9 ratchet connection ring, | 10 injection voicing ratchet, |
| 11 return spring, | 12 pen core holder connecting tube, |
| 13 pen cap, | 14 button spring, |
| 15 numerical code tube, | 16 button connecting member, |
| 17 adjusting tube, | 18 push rod, |
| 19 injection bouncing spring, | 20 positioning ratchet, |
| 21 positioning rotary ratchet, | 22 push rod nut, |
| 23 pusher head, | 24 pen core holder. |

### DETAILED DESCRIPTION OF THE EMBODIMENTS

As described in the background section, conventional injection pens are inconvenient to adjust the dose at the time of injection.

Based on the above research, an injection pen capable of fine adjustment is provided according to embodiments of the present application. When the dose before the injection is adjusted, a knob is rotated to drive a numerical code tube, a adjusting tube, a ratchet connecting ring, a ratchet tube, a button connecting member, and a button to simultaneously move upward, wherein the adjusting tube rotates along a screw lead, the adjusting tube and the ratchet connecting ring are assembled with each other by ratchets, and due to a reaction force from a button spring, there will be "click" sounds at a contact region of the ratchet connecting ring and the ratchet tube, and the adjusted dose can be observed through a window during the adjusting process. If the dose is too much, the dose can be readjusted by turning the knob. Since the ratchet connecting ring has ratchets at both ends, there will also be the "click" sounds at a contact region of the ratchet connecting ring and the adjusting tube in the adjusting process. The sounds can remind the user, and the visual prompt of the window can also be combined to facilitate the dose adjustment. Besides, the injection pen capable of fine adjustment can be reused, thereby avoiding use of conventional disposable pen consumables and reducing medical waste. Compared with the conventional direct-push injection pen, the injection precision can be effectively improved by converting a spiral motion into a linear motion.

In order to make the above objectives, features and advantages of the present application more apparent and easy to be understood, embodiments of the present application will be described in detail hereinafter in conjunction with the drawings.

Specific details are set forth in the following description for a better understanding of the present application. However, the present application may be embodied in many other ways than those described herein, and those skilled in the art may make similar improvements without departing from the essence of the present application. The present application is therefore not limited by the specific embodiments disclosed below.

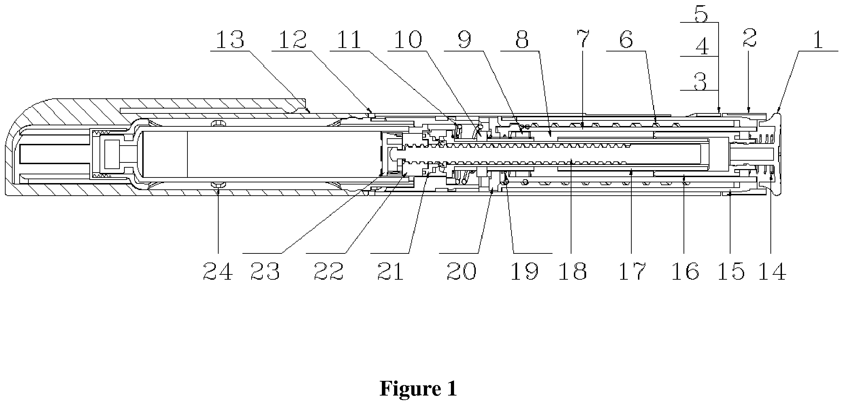

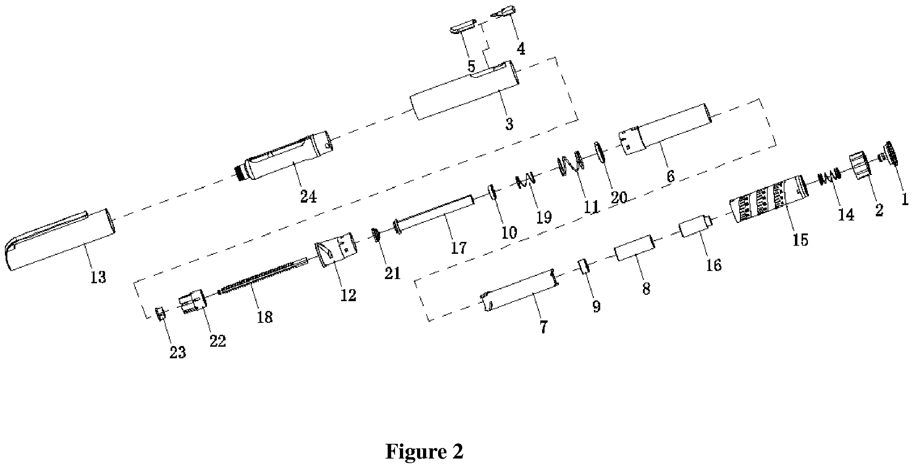

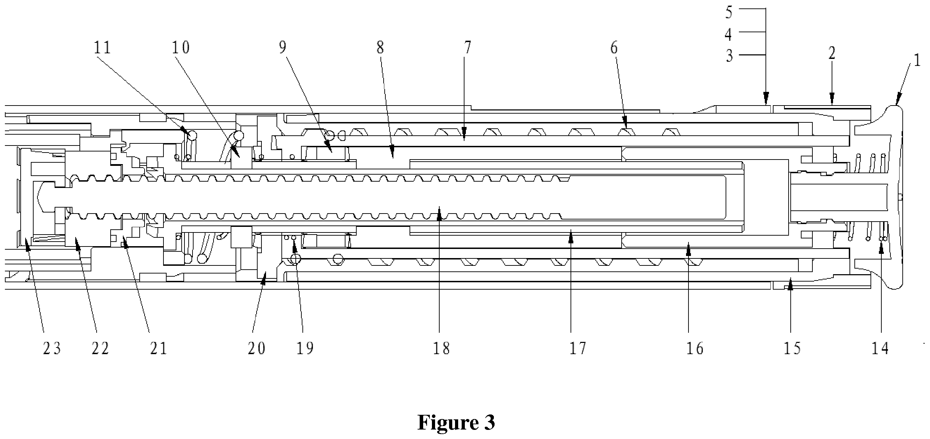

Referring to Figures 1 to 3, Figure 1 is a cross-sectional structural view of an injection pen capable of fine adjustment according to a specific embodiment of the present application; Figure 2 is a schematic exploded view of the injection pen capable of fine adjustment according to a specific embodiment of the present application; and Figure 3 is a partial cross-sectional structural view of the injection pen capable of fine adjustment according to a specific embodiment of the present application.

The injection pen capable of fine adjustment provided by the specific embodiment of the present application includes: a differential screw drive mechanism, including a push rod 18 for pushing a cork of a cartridge bottle, a push rod nut 22 screwed to the push rod 18, and a positioning rotary ratchet 21 axially abutting the push rod nut 22, an adjusting rod 17, one end of which is in a detachable connection with the positioning rotary ratchet 21, and a ratchet tube 8 which controls the rotation of the adjusting rod 17, in which the ratchet tube 8 is arranged around an outside of the adjusting rod 17; a dose adjustment mechanism, including a knob 2, a numerical code tube 15, an adjusting tube 7, a ratchet connecting ring 9, and an adjustment connecting tube 6, in which the ratchet connecting ring 9 is arranged around an outside of the adjusting rod 17, the adjustment connecting tube 6 is arranged around an outside of the adjusting rod 17, the ratchet connecting ring 9 and the ratchet tube 8, ratchets at two ends of the ratchet connecting ring 9 are inclined in a same direction, the two ends of the ratchet connecting ring 9 are respectively connected to the adjusting rod 17 and the ratchet tube 8 by the ratchets, the adjustment connecting tube 6 is in threaded connection with outside of the adjusting tube 6, the numerical code tube 15 is arranged around an outside of the adjustment connecting tube 6, and a top end of the numerical code tube 15 is snapped inside the knob 2, a tail end of the numerical code tube 15 is arranged around the outside of the adjusting tube 7, and an outer wall of the numerical code tube 15 is provided with scale marks distributed in a spiral shape; a housing 3, in which the differential screw drive mechanism is arranged in the housing 3, the housing 3 is provided with a window 4 for displaying a scale on the numerical code tube 15 and a pen core holder 24 for mounting the cartridge bottle; and an injection mechanism, including a button connecting member 16 which has one end in contact with one end of the ratchet tube 8, a button 1 which pushes the button connecting member 16 to move axially, and a button spring 14 which abuts the button connecting member 16 and the button 1 at two ends of the button spring 14.

As for the injection pen capable of fine adjustment according to the present embodiment, when the cartridge bottle is mounted into the pen core holder 24, the push rod nut 22 is forced to slide upwards and the positioning rotary ratchet 21 moves accordingly. After the cartridge bottle is fitted in position, the positioning rotary ratchet 21 engages with a ratchet at the end of the adjusting rod 17. The pen core holder 24 can be connected to the housing 3 through a pen core holder connecting tube 12, and a pen cap 13 is further arranged around an outside of the pen core holder 24.

When the dose before the injection is adjusted, the knob 2 is rotated to drive the numerical code tube 15, the adjusting tube 7, the ratchet connecting ring 9, the ratchet tube 8, the button connecting member 16, and the button 1 to simultaneously move upward, and the adjusting tube 7 rotates along a screw lead, the adjusting tube 7 and the ratchet connecting ring 9 are assembled with each other by ratchets, and due to a reaction force from the button spring 14, there will be "click" sounds at a contact region of the ratchet connecting ring 9 and the ratchet tube 8, and the adjusted dose can be observed through the window 4 during the adjusting process. If the dose is too much, the dose can be readjusted by turning the knob 2. Since the ratchet connecting ring 9 has ratchets at both ends, there will also be the "click" sounds at a contact region of the ratchet connecting ring 9 and the adjusting tube 7. The sounds can remind the user, and the visual prompt of the window 4 can also be combined to facilitate the dose adjustment.

After the dose is adjusted to a required amount, the injection can be performed: the button 1 is pressed, the button connecting member 16 moves linearly along its axial direction and drives the ratchet tube 8, the ratchet connecting ring 9, the adjusting tube 7 and the numerical code tube 15, the adjusting rod 17, and the positioning rotary ratchet 21 to move spirally, which in turn drives the push rod 18 to rotate. Under the action of the push rod nut 22, the push rod 18 moves axially, and then pushes the cork of the cartridge bottle to inject.

Besides, the injection pen capable of fine adjustment according to the above embodiment can be reused, thereby avoiding use of conventional disposable pen consumables and reducing medical waste. Besides, compared with the conventional direct-push injection pen, the injection precision can be effectively improved by converting the spiral motion into the linear motion.

Furthermore, a return spring 11 is provided in the housing 3 for pushing the push rod nut 22 to move downward and further driving the positioning rotary ratchet 21 and the adjusting rod 17 to be separated from each other. A bottom end of the return spring 11 closely abuts against an upper end of the push rod nut 22.

When the cartridge bottle is installed, the return spring 11 is contracted under the squeeze of the push rod nut 22; after the cartridge bottle is taken out, the push rod nut 22 is driven by the return spring 11 to move downward, and since the push rod nut 22 and the positioning rotary ratchet 21 are connected to each other, the positioning rotary ratchet 21 and the adjusting rod 17 can be separated from each other.

Furthermore, the housing 3 is further provided with a positioning ratchet 20. The positioning ratchet 20 is arranged around an outside of the adjusting rod 17, and an injection voicing ratchet 10 and an injection bouncing spring 19 are arranged around the outside of the adjusting rod 17. The injection voicing ratchet 10 butts the positioning ratchet 20 through the injection bouncing spring 19.

During the installation of the cartridge bottle, the adjusting rod 17 and the injection voicing ratchet 10 move linearly, and the injection voicing ratchet 10 come into abutting with the positioning ratchet 20 under the squeeze of the injection bouncing spring 19.

During the injection process, since the positioning ratchet 20 is fixed, there will be "click" sounds due to the rotation of the injection voicing ratchet 10, so as to give the user an auditory reminder about the injection.

In order to optimize the use effect of the push rod 18 provided by the above embodiment, a pusher head 23 is provided between the push rod nut 22 and the cork of the cartridge bottle. A chamber may be arranged in the pusher head 23, and a latching block may be arranged at an end of the push rod 18. The latching block is latched in the chamber, so that when the push rod 18 rotates, the pusher head 23 is not driven to rotate, and the pusher head 23 only moves axially.

Furthermore, axially distributed grooves are provided on an outside wall of the adjusting rod 17, and sliding blocks cooperating with the grooves are provided on an inside wall of the ratchet tube 8. The grooves facilitate the movement of the ratchet tube 8, and also help the ratchet tube 8 to drive the adjusting rod 17 to rotate. Two grooves may be symmetrically on the adjusting rod 17, and an inner ring of the injection voicing ratchet 10 is also provided with a sliding block adapted to the grooves, so that the adjusting rod 17 and the injection voicing ratchet 10 can rotate synchronously.

Furthermore, the window 4 is provided with a window cover 5. The scale on the numerical code tube 15 can be protected by the window cover 5 to increase its service life, and the window cover 5 is preferably transparent.

To be further noted, the term "include", "comprise" or any variant thereof is intended to indicate nonexclusive inclusion so that a process, method, article or device including a series of elements includes not only those explicitly described elements but also other elements which have not been listed explicitly or an element(s) inherent to the process, method, article or device. With no other limitations, an element restricted by the phrase "include a ..." does not exclude the existence of other identical elements in the process, method, item or apparatus including the element.

In the present specification, the embodiments are described in a progressive manner. Each embodiment mainly focuses on an aspect different from other embodiments, and reference can be made to these similar parts among the embodiments.

According to the above description of the disclosed embodiments, those skilled in the art can implement or practice the present application. Many changes to these embodiments are apparent for those skilled in the art, and general principles defined herein may be implemented in other embodiments without departing from the spirit or scope of the present application. Hence, the present application is not limited to the embodiments disclosed herein, but is to conform to the widest scope in accordance with the principles and novel features disclosed herein.

## Claims

1. An injection pen capable of fine adjustment, **characterized in that**, the injection pen comprises: a differential screw drive mechanism, a dose adjustment mechanism, a housing, and an injection mechanism,
the differential screw drive mechanism comprises a push rod configured to push a cork of a cartridge bottle, a push rod nut screwed to the push rod, and a positioning rotary ratchet axially abutting the push rod nut, an adjusting rod which is in a detachable connection with the positioning rotary ratchet at one end of the adjusting rod, and a ratchet tube which controls the rotation of the adjusting rod, and the ratchet tube is arranged around an outside of the adjusting rod;
the dose adjustment mechanism comprises a knob, a numerical code tube, an adjusting tube, a ratchet connecting ring, and an adjustment connecting tube, and the ratchet connecting ring is arranged around the outside of the adjusting rod, the adjustment connecting tube is arranged around an outside of the adjusting rod, the ratchet connecting ring and the ratchet tube, ratchets at two ends of the ratchet connecting ring are inclined in a same direction, two ends of the ratchet connecting ring are respectively connected to the adjusting rod and the ratchet tube by the ratchets, the adjustment connecting tube is in a threaded connection with the outside of the adjusting tube, the numerical code tube is arranged around the outside of the adjustment connecting tube, and a top end of the numerical code tube is snapped inside the knob, and an outer wall of the numerical code tube is provided with scale marks distributed in a spiral shape;
the differential screw drive mechanism is arranged in the housing, the housing is provided with a window configured to display a scale on the numerical code tube and a pen core holder configured to mount the cartridge bottle; and
the injection mechanism comprises a button connecting member which has one end in contact with one end of the ratchet tube, a button which pushes the button connecting member to move axially, and a button spring, two ends of which respectively abut the button connecting member and the button, and the button connecting member and the ratchet connecting ring move axially, and the numerical code tube move spirally in an axial direction, after the button is pressed.

2. The injection pen capable of fine adjustment according to claim 1, **characterized in that**, a return spring is provided in the housing, and is configured to push the push rod nut to move downward and further drive the positioning rotary ratchet and the adjusting rod to be separated from each other, and a bottom end of the return spring closely abuts against an upper end of the push rod nut.

3. The injection pen capable of fine adjustment according to claim 2, **characterized in that**, the housing is further provided with a positioning ratchet, the positioning ratchet is arranged around the outside of the adjusting rod, and an injection voicing ratchet and an injection bouncing spring are arranged around the outside of the adjusting rod, and the injection voicing ratchet butts the positioning ratchet through the injection bouncing spring.

4. The injection pen capable of fine adjustment according to claim 3, **characterized in that**, a pusher head is provided between the push rod nut and the cork of the cartridge bottle.

5. The injection pen capable of fine adjustment according to claim 4, **characterized in that**, axially distributed grooves are provided on an outside wall of the adjusting rod and sliding blocks cooperating with the grooves are provided on an inside wall of the ratchet tube.

6. The injection pen capable of fine adjustment according to claim 5, **characterized in that**, the window is provided with a window cover.
